# EUROPEAN PATENT APPLICATION

(11) **EP 1 430 911 A2**
(43) Date of publication of application: **23.06.2004**
(21) Application number: 03254119.5
(22) Date of filing: 27.06.2003
(51) Int. Cl.: A61L 15/28, A61L 27/20

(54) **Hemostatic wound dressing and fabric containing aldehyde-modified polysaccharide**

(30) Priority: 20.12.2002 US 326244
(71) Applicant: Ethicon, Somerville, NJ 08876 (US)
(72) Inventor: Zhang, Guanghui, Belle Mead, NJ 08502 (US); Pendharkar, Sanyog Manohar, Old Bridge, NJ 08857 (US); Guo, Jian Xin, Bridgewater, NJ 08807 (US); Gorman, Anne Jessica, Hightstown, NJ 08520 (US); Looney, Dwayne Lee, Flemington, NJ 08822 (US)
(74) Representative: Mercer, Christopher Paul

(57) **Abstract**

The present invention is directed to hemostatic wound dressings containing a fabric made from biocompatible, aldehyde-modified polysaccharide fibers; and a porous, polymeric matrix made from a biocompatible, water-soluble or water-swellable polymer, dispersed at least partially through the fabric, to methods of making such wound dressings and to methods of providing hemostasis to a wound.

## Description

### FIELD OF THE INVENTION

The present invention relates to hemostatic wound dressings containing or fabricated from a fabric comprising an aldehyde-modified polysaccharide, e.g. aldehyde-modified regenerated cellulose, and a porous water-soluble or water-swellable polymeric matrix, to a process of making such fabrics and wound dressings, and to a method of providing hemostasis to a wound.

### BACKGROUND OF THE INVENTION

The control of bleeding is essential and critical in surgical procedures to minimize blood loss, to reduce post-surgical complications, and to shorten the duration of the surgery in the operating room. Cellulose that has been oxidized to contain carboxylic acid moieties, i.e. oxidized cellulose (OC) due to its biodegradable, bactericidal, and hemostatic properties, has long been used as a topical hemostatic wound dressing in a variety of surgical procedures, including neurosurgery, abdominal surgery, cardiovascular surgery, thoracic surgery, head and neck surgery, pelvic surgery, and skin and subcutaneous tissue procedures.

The use of oxidized cellulose as a hemostat was first described by Virginia Franz in 1944. Currently available oxidized cellulose hemostats are knitted or non-woven fabrics comprising carboxylic oxidized cellulose. Oxidized regenerated cellulose (ORC) is carboxylic-oxidized cellulose comprising reactive carboxylic acid groups. Examples of ORC absorbable hemostats commercially available include Surgicel® absorbable hemostat, a knitted fabric of ORC; Surgicel Nu-Knit® absorbable hemostat, a dense ORC fabric; and Surgicel® Fibrillar absorbable hemostat; all available from Johnson & Johnson Wound Management Worldwide, a division of Ethicon, Inc., Somerville, New Jersey, a Johnson & Johnson Company. Other examples of commercial absorbable hemostats containing oxidized cellulose include Oxycel® absorbable cellulose surgical dressing from Becton Dickinson and company, Morris Plains, New Jersey.

Conventional oxidized cellulose (OC) and oxidized regenerated cellulose (ORC) hemostats noted above are knitted, woven or non-woven fabrics comprising carboxylic acid groups, as noted above. However, the acid-based ORC and OC, due to their acidic pH, also rapidly denature acid-sensitive, hemostatic proteins, including thrombin or fibrinogen, on contact. Thus, it is most problematic to use the OC or ORC as a carrier for acid-sensitive species, such as thrombin and fibrinogen, as well as other acid-sensitive biologics and pharmaceutical agents. In addition to issues concerning compatibility of conventional OC and ORC with "acid-sensitive" species, e.g. proteins, drugs, etc., while the absorbency of body fluid and the hemostatic action of such currently available oxidized cellulose hemostats are adequate for applications where mild to moderate bleeding is encountered, they are not known to be effective to prevent or stop severe bleeding of high volume and high blood flow rate where a relatively high volume of blood is lost at a relatively high rate, nor are they known to achieve rapid hemostasis. In such instances, e.g. arterial puncture, liver resection, blunt liver trauma, blunt spleen trauma, aortic aneurysm, bleeding from patients with over-anticoagulation, or patients with coagulopathies, such as hemophilia, etc., a higher degree of hemostasis is required quickly. In an effort to achieve enhanced hemostatic properties, blood-clotting agents, such as thrombin, fibrin and fibrinogen have been combined with other carriers or substrates for such agents, including gelatin-based carriers and a collagen matrix.

Hemostatic wound dressings containing neutralized OC and protein-based hemostatic agents, such as thrombin, fibrinogen and fibrin are known. Neutralized OC is prepared by treating the OC with a water or alcohol solution of a basic salt of a weak organic acid to elevate the pH of the OC to between 5 and 8 by neutralizing the acid groups on the OC prior to addition of thrombin in order to make it thrombin-compatible. While such neutralized OC may be thrombin compatible, it is no longer bactericidal, as the anti-microbial activity of the OC is due to its acidic nature.

Hemostatic agents such as thrombin, fibrinogen or fibrin, if not effectively bound chemically or physically to the substrate, may be rinsed away by blood at a wound site. The unbound agent may migrate into the blood stream, which is undesired.Methods of producing highly oxidized tri-carboxylic acid derivatives of cellulose as hemostatic materials, involving two-stage oxidation by successive processing with an iodine-containing compound and nitrogen oxides, has been disclosed in RU2146264 and IN159322. As disclosed in these disclosures, oxidized cellulosic materials were prepared by preliminary oxidation with metaperiodate or periodic acid to yield periodate-oxidized, dialdehyde cellulose to form the intermediate for forming OC. The dialdehyde cellulose intermediate then is further oxidized by NO₂ to yield the OC, which then is used as a hemostatic, anti-microbial and wound-healing agent. It would be advantageous to provide a hemostatic wound dressing that not only provides hemostasis and anti-microbial properties similar to conventional OC-containing hemostatic wound dressings, but that also is compatible with "acid-sensitive" species.

It also would be advantageous to provide an anti-microbial hemostatic wound dressing that not only exhibits improved hemostasis over conventional wound dressings, but that does so without the risk of hemostatic agents migrating into the blood stream.

The present invention provides such a wound dressing that not only provides hemostatic and anti-microbial properties equivalent to or better than conventional OC-based hemostatic wound dressings, but that also is compatible with "acid-sensitive" species.

### SUMMARY OF THE INVENTION

The present invention is directed to hemostatic wound dressings that contain a fabric. The fabric has a first wound-contacting surface and a second surface opposing the wound-contacting surface. The fabric comprises fibers and has flexibility, strength and porosity effective for use as a hemostat. The fibers are prepared from a biocompatible, aldehyde-modified polysaccharide. The wound dressing also contains a porous, polymeric matrix applied at least to the wound-contacting surface of and preferably dispersed at least partially through the fabric. The porous, polymeric matrix comprises a biocompatible, water-soluble or water-swellable polymer. The invention also is directed to methods of making such wound dressings and to methods of providing hemostasis to a wound that includes applying the wound dressing of the present invention to a wound.

### BRIEF DESCRIPTION OF THE FIGURES

Figure 1 is an image produced by scanning electron microscopy (X75) of a cross section of a comparative wound dressing ORC fabric.
Figure 2 is an image produced by scanning electron microscopy (X75) of the wound-contact surface of a comparative wound dressing ORC fabric.
Figure 3 is an image produced by scanning electron microscopy (X75) of a cross section of a fabric according to the present invention.
Figure 4 is an image produced by scanning electron microscopy (X75) of the wound-contact surface of a fabric according to the present invention.
Figure 5 is an image produced by scanning electron microscopy (X75) of a cross-section of a wound dressing of the present invention.
Figure 6 is an image produced by scanning electron microscopy (X75) of the wound-contact surface of a wound dressing of the present invention.
Figure 7 is an image produced by scanning electron microscopy (X75) of the top surface of a wound dressing of the present invention.

### DETAILED DESCRIPTION OF THE INVENTION

The present invention is directed to fabrics and hemostatic wound dressings fabricated at least in part from such fabrics, and to methods of making and using the wound dressings.

Wound dressings of the present invention comprise a fabric that comprises fibers prepared from a biocompatible, aldehyde-modified polysaccharide, preferably a biodegradable, polysaccharide. The fabric includes a first wound-contacting surface, and a second surface opposing the first surface. The fabric preferably possesses physical properties suitable for use as a hemostat, including flexibility, strength and porosity.

The wound dressing further includes a porous, biocompatible, water-soluble or water-swellable polymeric matrix applied to the first surface of and dispersed at least partially through the fabric. In certain embodiments, the polymeric matrix will be dispersed substantially homogenously through the fabric.

In certain embodiments of the invention, the wound dressings will further include a hemostatic agent. The agent may be bound within the polymeric matrix, as well as to the first fabric surface and/or within the fabric. The agents may be bound by chemical or physical means, provided that they are bound such that they do not migrate from the wound dressing upon contact with blood in the body. As with the polymeric matrix, the hemostatic agent may be dispersed partially or homogenously through the fabric and/or the polymeric matrix. Preferably, the hemostatic agent is present in amounts effective to provide the wound dressings with the ability to provide and maintain effective hemostatis when applied to a wound in need of hemostasis.

The hemostatic wound dressings of the present invention provide and maintain effective hemostasis when applied to a wound requiring hemostasis. Effective hemostasis, as used herein, is the ability to control and/or abate capillary, venous, or arteriole bleeding within an effective time, as recognized by those skilled in the art of hemostasis. Further indications of effective hemostasis may be provided by governmental regulatory standards and the like.

The hemostatic dressings of the present invention are particularly useful when conventional procedures to control and/or abate bleeding, such as pressure or suturing, are either ineffective or impractical. In addition, the hemostatic wound dressings of the present invention may be used with hemostatic agents, or other biological or therapeutic compounds, moieties or species, that are "acid-sensitive", meaning that they may be degraded or denatured by, or otherwise detrimentally affected by acidic pH, such as is provided by conventional OC hemostatic wound dressings.

In certain embodiments of the invention, the fabrics utilized in the present invention may be knitted, woven or non-woven, provided that the fabric possesses the physical properties adequate for wound dressings, in general, and preferably for hemostatic wound dressings. A preferred woven fabric has dense and knitted structure that provides form and shape for the hemostatic wound dressing. Fabrics oxidized by periodic acid or its salts described in the present invention are expected to retain physical properties and mechanical integrity required for use in wound dressings. Fabrics useful in hemostatic wound dressings according to the present invention include fabrics comprising the aldehyde-modified polysaccharides of the present invention and being of the structure described in United States Patent Number 4,626,253, the contents of which is hereby incorporated by reference herein as if set forth in its entirety.

In certain embodiments of the invention, the hemostatic wound dressing of the present invention comprise a warp knitted tricot fabric constructed of bright rayon yarn that has been oxidized by periodic acid or its salts such that the comprises aldehyde moieties. Both Scanning Electron Microscopic (SEM) images and fabric mechanical properties indicate that the physical characteristics (density, thickness) and physical performance, e.g. fabric tensile strength and Mullen burst strength, of the aldehyde-modified regenerated cellulose fabric used in the present invention are comparable to those of the fabric disclosed in United States Patent 4,626,253.

The hemostatic dressing of the present invention remains very flexible, conforms to a bleeding site, and retains good tensile and compressive strength to withstand handling during application. The aldehyde-modified regenerated cellulose fabric and wound dressings can be cut into different sizes and shapes to fit the surgical needs. It can be rolled up or packed into irregular anatomic areas.

Other warp knit tricot fabric constructions that produce equivalent physical properties may, of course, be utilized in the manufacture of the aldehyde-modified regenerated cellulose hemostatic wound dressings of the present invention. Such constructions will be apparent to those skilled in the art once having the benefit of this disclosure.

Fabrics utilized in wound dressings of the present invention comprise a biocompatible, aldehyde-modified polysaccharide. In preferred wound dressings, the polysaccharide will contain an amount of aldehyde moieties effective to render the modified polysaccharide biodegradable, meaning that the polysaccharide is degradable by the body into components that either are resorbable by the body, or that can be passed readily by the body. More particularly, the biodegraded components do not elicit permanent chronic foreign body reaction because they are absorbed by the body, such that no permanent trace or residual of the component is retained at the implantation site.

Aldehyde-modified polysaccharides used in the present invention include, without limitation, cellulose, cellulose derivatives, e.g. alkyl cellulose, for instance methyl cellulose, hydroxyalkyl cellulose, alkylhydroxyalkyl cellulose, cellulose sulfate, salts of carboxymethyl cellulose, carboxymethyl cellulose and carboxyethyl cellulose, chitin, carboxymethyl chitin, hyaluronic acid, salts of hyaluronic acid, alginate, alginic acid, propylene glycol alginate, glycogen, dextran, dextran sulfate, curdlan, pectin, pullulan, xanthan, chondroitin, chondroitin sulfates, carboxymethyl dextran, carboxymethyl chitosan, heparin, heparin sulfate, heparan, heparan sulfate, dermatan sulfate, keratin sulfate, carrageenans, chitosan, starch, amylose, amylopectin, poly-N-glucosamine, polymannuronic acid, polyglucuronic acid, polyguluronic acid and derivatives of the above. In preferred embodiments, the polysaccharide is oxidized as described herein to assure that the aldehyde-modified polysaccharide is biodegradable.

Biodegrable, aldehyde-modified, regenerated polysaccharides used in the present invention may be represented by Structure I below. 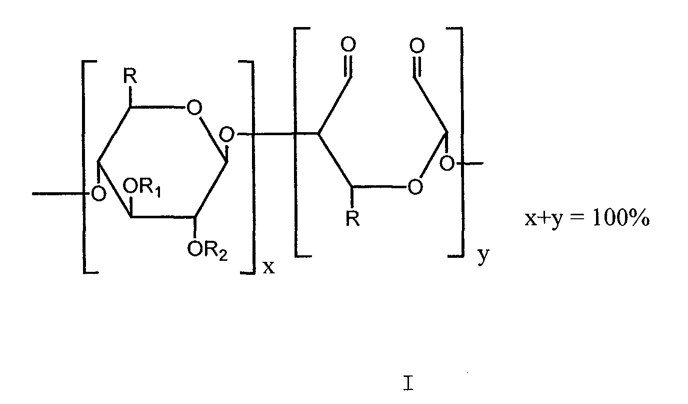 where x and y represent mole percent, x plus y equals 100 percent, x is from about 95 to about 5,
y is from about 5 to about 95; and
R may be CH₂OR₃, COOR₄, sulphonic acid, or phosphonic acid; R₃ and R₄ may be H, alkyl, aryl, alkoxy or aryloxy, and R₁ and R₂ may be H, alkyl, aryl, alkoxy, aryloxy, sulphonyl or phosphoryl.

In preferred embodiments of the present invention, the fabric is prepared from a biocompatible, biodegradable, aldehyde-modified, regenerated polysaccharide. Regenerated cellulose is preferred due to its higher degree of uniformity versus cellulose that has not been regenerated. Regenerated cellulose is described in, for instance, United States Patent 3,364,200, the contents of which is hereby incorporated by reference as if set forth in its entirety.

In particular, preferred aldehyde-modified regenerated cellulose used in the present invention comprises repeating units of Structure II below: 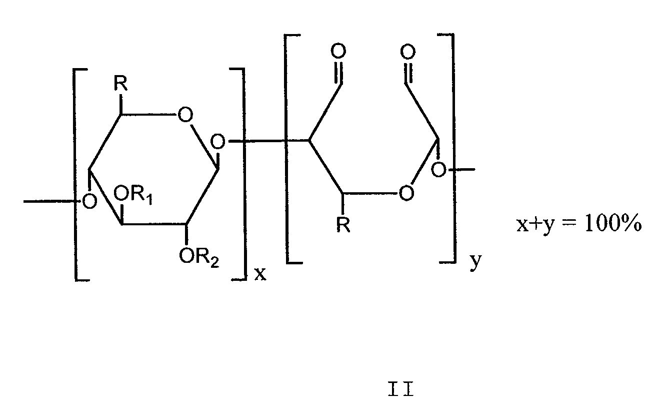 where x and y represent mole percent, x plus y equals 100 percent, x is from about 95 to about 5,
y is from about 5 to about 95; R is CH₂OH, and R₁ and R₂ are H.

In certain embodiments of the present invention, x is from about 90 to about 10 and y is about 10 to about 90. Preferably, x is from about 80 to about 20 and y is from about 20 to about 80. Even more preferably, x is from about 70 to about 30. Most preferably, x is about 70 and y is about 30.

The fabric and hemostatic wound dressings of the present invention also provide anti-microbial activity due to the presence of effective amounts of the aldehyde moieties. It has been shown that in spite of being essentially free of acidic groups, the aldehyde-modified regenerated cellulose is anti-microbial in nature, meaning that the fabric and dressing substantially inhibit colonization of certain microorganisms on or near the fabric and dressing. The hemostats of the present invention were found to be significantly effective against microorganisms, such as Methicillin-resistant *Staphylococcus aureus* (MRSA) *and Pseudomonas aeruginosa*, etc. The anti-microbial activity of the non-acidic, aldehyde-modified regenerated cellulose is shown to be comparable to that of the acidic, carboxylic oxidized regenerated cellulose (ORC) conventionally used. However, the aldehyde-modified regenerated cellulose utilized in the present invention is expected to retain its anti-microbial activity over a longer period of time, while conventional ORC loses its anti-microbial activity over a period of time as the acid groups are neutralized in the body.

In preferred embodiments of the invention, the aldehyde-modified regenerated polysaccharide, e.g. cellulose, is essentially free of functional or reactive moieties other than aldehyde moieties. By essentially free, it is meant that the polysaccharide does not contain such functional or reactive moieties in amounts effective to alter the properties of the aldehyde-modified polysaccharide or to provide the fabric comprising the polysaccharide with a pH of less than about 4.5, more preferably less than about 5, or greater than about 9, preferably about 9.5. Such moieties include, without limitation, carboxylic acid moieties typically present in wound dressings made from OC. Excess levels of carboxylic acid moieties will lower the pH of the fabrics and dressings so that they are not compatible for use with those acid sensitive species that may be degraded or denatured by such a low pH, e.g. thrombin. Other moieties essentially excluded include, without limitation, sulfonyl or phosphonyl moieties.

The fabric used in the present invention exhibits increased thermal stability compared to those of the carboxylic oxidized regenerated cellulose fabric (ORC) or neutralized ORC.

The wound dressing of the present invention comprise a porous, polymeric matrix. A preferred method of making the porous, polymeric matrix is to contact the fabric with an appropriate amount of a solution of a water-soluble or water-swellable polymer in an appropriate solvent therefore, thereby dispersing the dissolved polymer on the wound-contacting surface of and at least partially through the fabric, flash-freeze the polymer and fabric, thereby immobilizing the polymeric matrix, and then remove the solvent from the frozen structure under vacuum. Through this preferred lyophilization method, a fabric comprising a matrix of the water-soluble or water-swellable polymer having microporous or nanoporous structure is obtained. The lyophilization condition is important to the novel porous structure in order to create a large surface area in the hemostat with which body fluids can interact.

The features of such microporous structure can be controlled to suit a desired application by choosing the conditions to form the composite hemostat during lyophilization. To maximize the surface area of the porous matrix according to the present invention, a preferred method is to quickly freeze the fabric/polymer construct at lower than 0°C, preferably at about -50°C, and to remove the solvent under high vacuum. The porous matrix produced thereby provides a large fluid absorbing capacity to the hemostatic wound dressing. When the hemostatic wound dressing comes into contact with body fluid, a very large surface area of polymer is exposed to the fluid instantly. The hydration force of the fabric and subsequent formation of a tacky gelatinous layer helps to create an adhesive interaction between the wound dressing and the bleeding site. The microporous structure of the polymeric matrix also allows blood to quickly pass through the fabric surface before the hydration takes place. The formation of a gelatinous sheet on aldehyde-modified cellulose fabric upon blood contact will enhance the sealing property of the water-soluble gelatinous layer, which is critical to fast hemostasis for surgical bleeding.

The wound dressing comprises the polymeric matrix dispersed on and within the fabric in an amount effective to provide and maintain effective hemostasis in cases of surgical bleeding. If the ratio of polymer to fabric is too low, the polymer does not provide an effective seal to physically block the bleeding. If the ratio is too high, the composite hemostat wound dressing will be too stiff or too brittle to conform to wound tissue in surgical applications. Such an excessive ratio will also prevent the blood from quickly passing through the matrix to the fabric surface to form the gelatinous layer that is critical for enhancing the sealing property. A preferred weight ratio of polymer to fabric is from about 1:99 to about 15:85. A more preferred weight ratio of polymer to fabric is from about 3:97 to about 10:90.

In certain embodiments of the present invention, the porous, polymeric matrix is dispersed substantially homogeneously on at least the wound-contacting surface of the fabric and through the fabric. In such cases, the fabric may be emersed in the polymer solution to provide homogeneous distribution throughout the fabric prior to lyophilization. In other embodiments, it may be preferred that only the wound-contact surface of the hemostat sticks well to wet surfaces, while the physician handling side, or top surface of the fabric, does not. In such cases, the fabric may be partially emersed in the polymer solution so as to provide polymer at least on the wound-contact surface of the fabric. In this way, a gradient of polymer in the fabric is provided, whereby the fabric will comprise an effective amount of the lyophilized polymer adjacent the wound-contacting area, while the top surface of the fabric comprises little or no dispersed polymer and maintains ease of handling for the physician.

The polymer used in the porous matrix of the present invention is a biocompatible, water-soluble or water-swellable polymer. The water-soluble or water-swellable polymer rapidly absorbs blood or other body fluids and forms a tacky or sticky gel adhered to tissue when placed in contact therewith. The fluid-absorbing polymer, when in a dry or concentrated state, interacts with body fluid through a hydration process. Once applied in a bleeding site, the polymer interacts with the water component in the blood via the hydration process. The hydration force provides an adhesive interaction that aids the hemostat adhere to the bleeding site. The adhesion creates a sealing layer between the hemostatic dressing and the bleeding site to stop the blood flow.

Polymers useful in polymeric matrices used in wound dressings of the present invention include, without limitation, polysaccharides, polyacrylic acids, polymethacrylic acids, polyamines, polyimines, polyamides, polyesters, polyethers, polynucleotides, polynucleic acids, polypeptides, proteins, poly (alkylene oxides), polythioesters, polythioethers, polyvinyls, polymers comprising lipids and derivatives of the above.

In preferred embodiments, the polymer comprises a water-soluble or water-swellable polysaccharide, preferably selected from the group consisting of cellulose, cellulose derivatives, e.g. alkyl cellulose, for instance methyl cellulose, hydroxyalkyl cellulose, alkylhydroxyalkyl cellulose, cellulose sulfate, salts of carboxymethyl cellulose, carboxymethyl cellulose and carboxyethyl cellulose, chitin, carboxymethyl chitin, hyaluronic acid, salts of hyaluronic acid, alginate, alginic acid, propylene glycol alginate, glycogen, dextran, dextran sulfate, curdlan, pectin, pullulan, xanthan, chondroitin, chondroitin sulfates, carboxymethyl dextran, carboxymethyl chitosan, heparin, heparin sulfate, heparan, heparan sulfate, dermatan sulfate, keratin sulfate, carrageenans, chitosan, starch, amylose, amylopectin, poly-N-glucosamine, polymannuronic acid, polyglucuronic acid, polyguluronic acid and derivatives of the above. Most preferred are sodium carboxymethyl cellulose, methyl cellulose, hydroxyethylcellulose and their aldehyde modified derivatives.

Water-soluble or water-swellable polymers used in other embodiments of the present invention where acid-sensitive agents may be utilized preferably comprise a non-acidic, water-soluble or water-swellable polysaccharide, preferably selected from the group consisting of methyl cellulose, hydroxyalkyl cellulose, water-soluble chitosan, salts of carboxymethyl cellulose, carboxyethyl cellulose, chitin, salts of hyaluronic acid, alginate, propylene glycol alginate, glycogen, dextran, carrageenans, chitosan, starch, amylose, poly-N-glucosamine and aldehyde modified derivatives of the above. Most preferred are sodium carboxymethyl cellulose, methyl cellulose, hydroxyethylcellulose and derivatives of the above, including aldehyde-modified derivatives.

In certain embodiments of the invention, a biologics, a drug, a hemostatic agent, a pharmaceutical agent, or combinations thereof, that otherwise may be sensitive to the low pH of conventional OC-containing wound dressings, may be incorporated into wound dressings of the present invention without having to adjust pH prior to incorporation into the dressing. To fabricate such a hemostatic wound dressing, a drug or agent may be dissolved in an appropriate solvent. The fabric may then be coated with the drug solution and the solvent removed. Preferred biologics, drugs and agent include analgesics, anti-infective agents, antibiotics, adhesion preventive agents, pro-coagulants, and wound healing growth factors.

As noted above, wound dressings of the present invention provide rapid hemostasis and maintain effective hemostasis in cases of severe bleeding. Examples of severe bleeding include, without limitation, arterial puncture, liver resection, blunt liver trauma, blunt spleen trauma, aortic aneurysm, bleeding from patients with over-anticoagulation, or bleeding from patients with coagulopathies, such as hemophilia. Hemostatic agents that may be used in wound dressings according to the present invention include, without limitation, procoagulant enzymes, proteins and peptides, can be naturally occurring, recombinant, or synthetic, and may be selected from the group consisting of prothrombin, thrombin, fibrinogen, fibrin, fibronectin, heparinase, Factor X/Xa, Factor VII/VIIa, Factor IX/IXa, Factor XI/XIa, Factor XII/XIIa, tissue factor, batroxobin, ancrod, ecarin, von Willebrand Factor, collagen, elastin, albumin, gelatin, platelet surface glycoproteins, vasopressin and vasopressin analogs, epinephrine, selectin, procoagulant venom, plasminogen activator inhibitor, platelet activating agents, synthetic peptides having hemostatic activity, derivatives of the above and any combination thereof. Preferred hemostatic agents used in the present invention are thrombin, fibrinogen and fibrin.

Protein-based hemostatic agents, such as thrombin, fibrin or fibrinogen, if bound to the wound dressing, can enhance the hemostatic property of aldehyde-modified regenerated cellulose wound dressings and reduce the risk of thrombosis caused by free hemostatic agents migrating into the blood stream. Hemostatic agents may be bound to the wound dressings either by chemical of physical means. Agents may be covalently conjugated with aldehyde groups pendant from the polysaccharide in one instance, thus chemically binding the agent to the wound dressing. Preferably, the hemostatic agents are physically bound to the wound dressing via incorporation into the polymeric matrix dispersed on and through the aldehyde-modified polysaccharide fabric and immobilized, i.e. bound, via lyophilization.

The hemostatic wound dressing of the present invention comprises hemostatic agents, including but not limited to thrombin, fibrinogen or fibrin, in an amount effective to provide rapid hemostasis and maintain effective hemostasis in cases of severe bleeding. If the concentration of the hemostatic agent in the wound dressing is too low, the hemostatic agent does not provide an effective proagulant activity to promote rapid clot formation upon contact with blood or blood plasma. A preferred concentration range of thrombin in the wound dressing is from about 0.001 to about 1 percent by weight. A more preferred concentration of thrombin in the wound dressing is from about 0.01 to about 0.1 percent by weight. A preferred concentration range of fibrinogen in the wound dressing is from about 0.1 to about 50 percent by weight. A more preferred concentration of fibrinogen in the wound dressing is from about 2.5 to about 10 by weight. A preferred concentration range of fibrin in the wound dressing is from about 0.1 to about 50 percent by weight. A more preferred concentration of fibrin in the wound dressing is from about 2.5 to about 10 by weight.

In certain embodiments, fabrics used in wound dressings of the present invention may comprise covalently conjugated there with a hemostatic agent bearing an aldehyde reactive moiety. In such embodiments, the aldehyde moiety of aldehyde-modified regenerated polysaccharide can readily react with the amine groups present on the amino acid side chains or N-terminal residues of thrombin, fibrinogen or fibrin, resulting in forming a conjugate of the hemostatic agent with the aldehyde-modified regenerated polysaccharide covalently linked by a reversible imine bond. The imine bonded aldehyde-modified regenerated polysaccharide/hemostatic agent conjugate may then be further reacted with a reducing agent such as sodium borohydride or sodium cyanoborohydride to form an irreversible secondary amine linkage. In such embodiments of the invention, the hemostatic agent is dispersed at least on the wound-contacting surface of the fabric, and preferably at least partially through the fabric structure, bound reversibly or irreversiblly to the aldehyde-modified polysaccharide.

Oxidation of 2, 3- vicinal hydroxyl groups in a carbohydrate with periodic acid (or any alkali metal salt thereof) forms a di-aldehyde or di-aldehyde derivatives. These aldehyde moieties(-RCH(O)) can then readily react with a primary amine moiety (-NH₂), such as are present on the amino acid side chains or N-terminal residues of proteins, resulting in an equilibrium with the reaction product, a protein and carbohydrate conjugate, covalently linked by a relatively unstable and reversible imine moiety (-N=CHR). To stabilize the linkage between the biomolecule and the substrate surface, subsequent reductive alkylation of the imine moiety is carried out using reducing agents (i.e., stabilizing agents) such as, for example, sodium borohydride, sodium cyanoborohydride, and amine boranes, to form a secondary amine (-NH-CH₂-R).

The features of such hemostatic agents conjugated with the aldehyde-modified regenerated cellulose wound dressing can be controlled to suit a desired application by choosing the conditions to form the composite hemostat during conjugation.

In such embodiments of the present invention, the hemostatic agent, such as thrombin, fibrinogen or fibrin, is dispersed substantially homogeneously through the wound dressing fabric. In such cases, aldehyde-modified regenerated cellulose fabric may be immersed in the solution of thrombin, fibrinogen or fibrin to provide homogeneous distribution throughout the wound dressing.

In certain embodiments of the invention, the thrombin conjugate of aldehyde-modified regenerated cellulose fabric is further reacted with reducing agents such as sodium borohydride or sodium cyanoborohydride to form a secondary amine linkage. The aldehyde-modified regenerated cellulose fabric can be soaked with the desired amount of aqueous solution of thrombin, then reacted with aqueous solution of sodium borohydride or sodium cyanoborohydride reconstituted in phosphate buffer (PH=8) prior to lyophilization.

The reduced form of the aldehyde-modified regenerated cellulose-thrombin conjugate is more stable due to the nature of the secondary amine linkage. Hemostatic wound dressings of this embodiment have enhanced hemostatic properties, as well as increased stability, and can provide rapid hemostasis without causing thrombin to migrate into the blood stream and cause severe thrombosis.

In preferred embodiments of the present invention, the hemostatic agent, such as thrombin, fibrinogen, or fibrin is constituted in an aqueous solution of a non-acidic, water-soluble or water-swellable polymer, as described herein above, including but not limited to methyl cellulose, hydroxyalkyl cellulose, water-soluble chitosan, salts of carboxymethyl carboxyethyl cellulose, chitin, salts of hyaluronic acid, alginate, propylene glycol alginate, glycogen, dextran, carrageenans, chitosan, starch, amylose, poly-N-glucosamine, and the aldehyde-modified derivatives thereof. The aldehyde-modified regenerated cellulose fabric can be soaked with the desired amount of aqueous solution of hemostatic agent and the water-soluble or water-swellable polymer and rapidly lyophilized using known methods that retain therapeutic activity. When constructed thusly, the hemostatic agent will be substantially homogenously dispersed through the polymeric matrix formed during lyophilization.

One skilled in the art, once having the benefit of this disclosure, will be able to select the appropriate hemostatic agent, water-soluble or water-swellable polymer and solvent therefore, and levels of use of both the polymer and hemostatic agent, depending on the particular circumstances and properties required of the particular wound dressing.

The present invention is best exemplified in the figures prepared by scanning electron microscope. The samples were prepared by cutting 1cm² sections by using a razor. Micrographs of both top surface and wound-contacting surfaces and cross-sections were prepared and mounted on carbon stubs using carbon paint. The samples were gold-sputtered and examined by scanning electron microscopy (SEM) under high vacuum at 4KV.

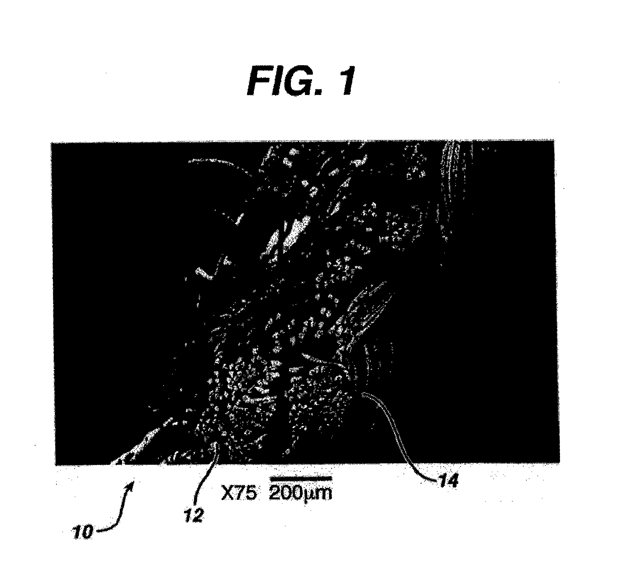

Conventional fabrics and fabrics according to the present invention are represented in Figures 1-4.

Figure 1 is a cross-section view (75X) of uncoated ORC fibers 12 organized as fiber bundles 14 and knitted into fabric 10 according to processes used conventionally to prepare such comparative fabrics. One commercial example of such a fabric is Surgicel Nu-Knit® absorbable hemostatic wound dressing.

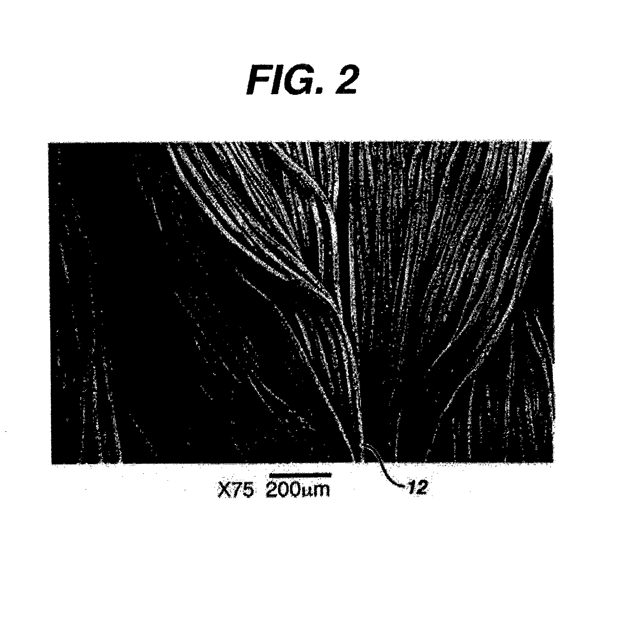

Figure 2 is a view of the wound-contact surface of the fabric of Figure 1. Individual fibers 12 are shown within a bundle.

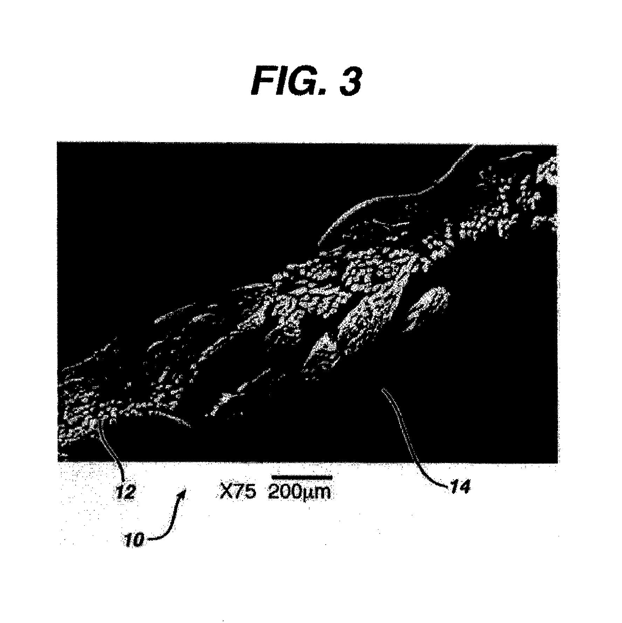

Figure 3 is a cross-section view (75X) of uncoated Aldehyde-Modified Regenerated Cellulose (AMRC) fibers 12 organized as fiber bundles 14 and knitted into fabric 10 according to preferred embodiments of the invention discussed herein above.

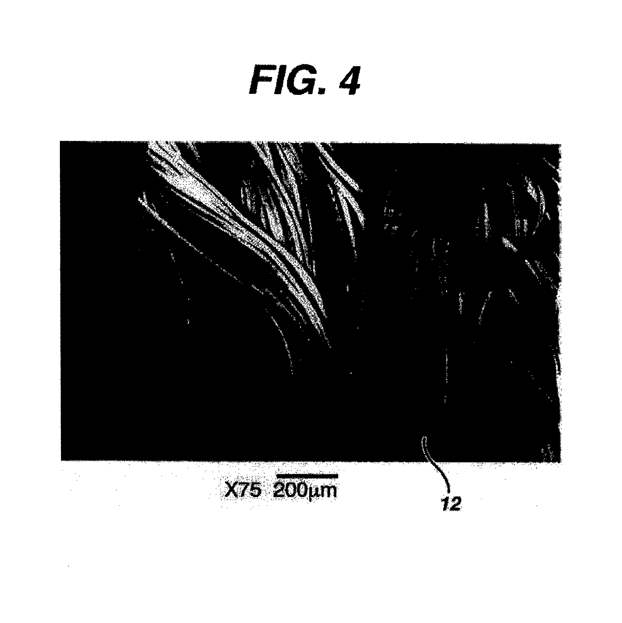

Figure 4 is a view of the wound-contact surface of the AMRC fabric of Figure 3. Individual fibers 12 are shown within a bundle.

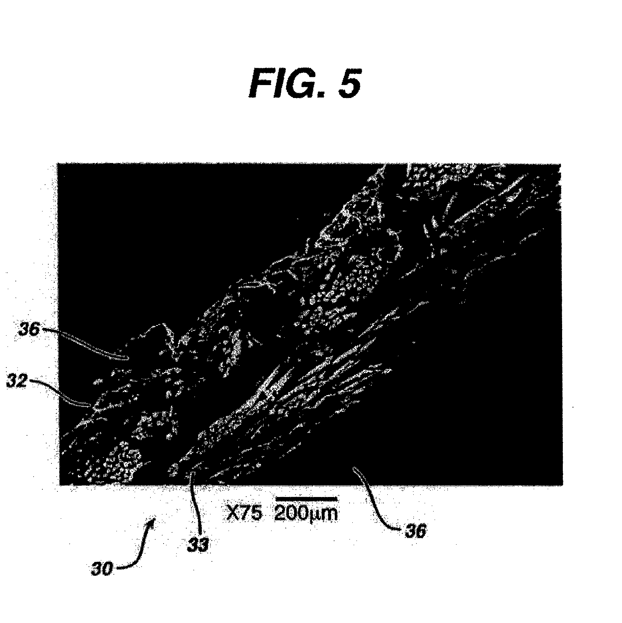

Hemostatic wound dressings according to the present invention are represented in Figures 5-7.

As shown in Figure 5, a porous, polymer matrix is substantially uniformly distributed on wound-contact surface 32 and throughout fabric 30. Polymer 36 forms a porous polymer matrix integrated with the knitted fibers 33. The porous, polymer matrix exhibits significant liquid absorption properties from capillary action in the same manner as a sponge.

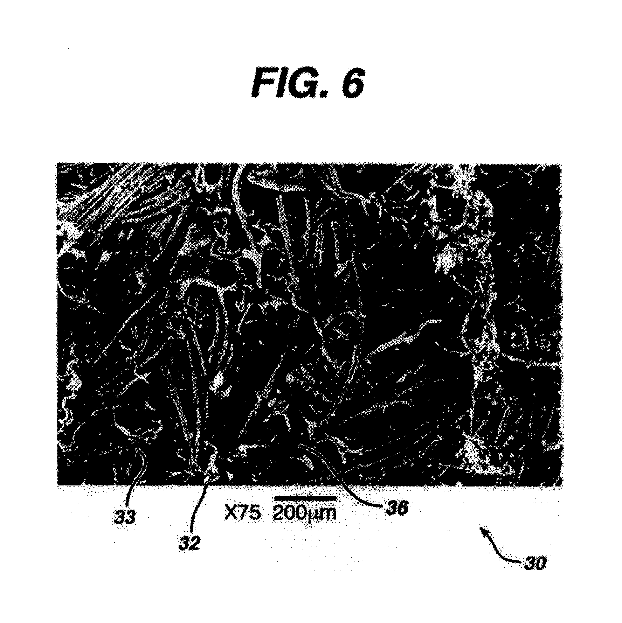

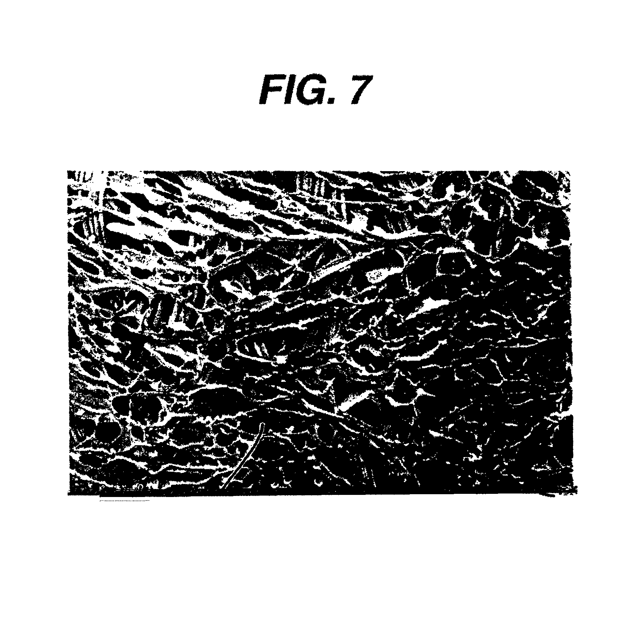

As shown in Figures 6 and 7, the polymer matrix disposed on the relative surfaces contains countless pores, ranging from about ten microns to as large as about 400 microns in diameter, or greater. Figure 6 shows wound-contact surface 32 of fabric 30. As noted, polymer 36 is present in the form of a porous matrix about fibers 33, thereby providing ample polymer surface area with which body fluids can interact upon contact therewith. Top surface 34 shown in Figure 7 also contains polymer 36 in the form of a porous matrix dispersed about fibers 33, thereby generating a sponge-like polymer matrix structure in concert with the fibers.

It is clear from Figures 5-7 that fabrics and wound dressings of the present invention contain a porous polymeric matrix dispersed on the wound-contact surface and substantially homogeneously through the fabric. Due to the porous nature of the matrix, body fluids are permitted to pass into the matrix, where ample surface area of polymer is present to interact with the body fluids. This results in faster and a higher degree of hemostasis.

It is clear hemostatic fabrics according to the present invention set forth in Figures 3-4 are of comparable construction, appearance and size compared to conventional hemostatic fabrics shown in Figures 1-2.

While the following examples demonstrate certain embodiments of the invention, they are not to be interpreted as limiting the scope of the invention, but rather as contributing to a complete description of the invention. Treatment times and temperatures for reactions in the examples below tend to be inversely related. Higher temperatures require relatively shorter treatment times. The limitations of the time and temperature are governed by the effect on the biological stability of the hemostatic agents.

### Example 1:

### Preparation of knitted aldehyde-modified regenerated (AMRC) cellulose fabric:

A 15.8 g piece of Nu-Knit® rayon fabric was cut in the form of a strip 1.5 inches wide. The strip was wound on a mandrel and suspended in 600 ml of aqueous isopropyl alcohol (IPA) (200 ml IPA/400 ml de-ionized (DI) water). 20.8 g of sodium periodate (Aldrich, Milwaukee, 53201) was dissolved in the solution (1:1 molar ratio) and the mandrel was rotated at moderate rpm in the solution for 21 hours at ambient temperature. It is essential that the oxidation of the fabric be conducted in the dark. The solution pH was 3.8. The solution was discarded after the reaction. The mandrel with the oxidized fabric was washed for 30 minutes in 1 liter of cold DI water containing 50 ml of ethylene glycol. It was then washed with aqueous IPA (50/50) for 15 minutes, followed by a pure IPA wash for 15 minutes. The fabric was dried in ambient air for several hours.

The oxidized fabric then was evaluated for hemostasis as set forth below. Results are provided in Table 1.

### Example 2:

### Preparation of water-soluble aldehyde-modified methylcellulose:

100 g of a 5% methylcellulose (MC, Ave. Mn 63kD, lot# 06827ES from Aldrich, Milwaukee, WI) aqueous solution was combined with 3 g of periodic acid (Aldrich, Milwaukee, 53201) and was then stirred for 5 hours at ambient temperature in the dark. 1.5 ml of ethylene glycol was added to the reaction solution and stirred for 30 minutes. 2000 ml of acetone were added slowly into the reaction solution to precipitate the aldehyde-modified methylcellulose (AMMC). The reaction mixture was allowed to stand for 20-30 minutes to separate the liquid phase from the solid phase. The supernatant then was removed and the solid phase centrifuged to precipitate the solids. The solid precipitate was dissolved in 100 ml DI over night followed by dialysis for 72 hours. The final wet mixture was lyophilized to form a sponge/foam.

### Example 3:

### Preparation of water-soluble aldehyde-modified hydroxyethyl cellulose:

100 g of a 5% hydroxyethyl cellulose (HEC, Ave. Mv; 720kD lot # 02808DU from Aldrich, Milwaukee, WI) aqueous solution was combined with 3 g of periodic acid (Aldrich, Milwaukee, 53201) and was then stirred for 5 hours at ambient temperature in the dark. 1.5 ml of ethylene glycol was added to the reaction solution and stirred for 30 minutes. 2000 ml of acetone were added slowly into the reaction solution to precipitate the aldehyde-modified hydroxyethyl cellulose. The reaction mixture was allowed to stand for 20-30 minutes to separate the liquid phase from the solid phase. The supernatant then was removed and the solid phase centrifuged to precipitate the solids. The solid precipitate was dissolved in 100 ml DI over night followed by dialysis for 72 hours. The final wet mixture was lyophilized to form a sponge/foam.

### Example 4:

### Aldehyde-modified regenerated cellulose (AMRC)/HEC porous patch preparation:

One gram of hydroxyethyl cellulose (HEC, Lot # GI01 from TCI, Tokyo, Japan) was dissolved in 99 grams of deionized water. After complete dissolution of the polymer, 10 grams of the HEC solution was transferred into a crystallization dish with a diameter of 10 cm. A piece of AMRC fabric (about 1.3 gram) was placed on the HEC solution in the crystallization dish. After soaking the fabric in the solution for 3 minutes, the wet fabric in the dish was lyophilized overnight. A very flexible patch was formed. The patch was further dried at room temperature under vacuum.

The AMRC/HEC patch then was evaluated for hemostasis as set forth below. Results are provided in Table 1.

### Example 5:

### AMRC/CS porous patch preparation

One gram of cellulose sulfate (CS, lot # A013801301 from ACROS Organics, New Jersey) was dissolved in 99 grams of deionized water. After complete dissolution of the polymer, 10 grams of the CS solution was transferred into a crystallization dish with a diameter of 10 cm. A piece of AMRC fabric (about 1.3 gram) was placed on the CS solution in the crystallization dish. After soaking the fabric for 3 minutes, the wet fabric was lyophilized overnight. A very flexible patch was formed. The patch was further dried at room temperature under vacuum.

The AMRC/CS patch then was evaluated for hemostasis as set forth below. Results are provided in Table 1.

### Example 6:

### AMRC/MC porous patch preparation

One gram of methyl cellulose (MC, Ave. Mn 63kD, lot# 06827ES from Aldrich, Milwaukee, WI) was dissolved in 99 grams of deionized water. After complete dissolution of the polymer, 10 grams of the MC solution was transferred into a crystallization dish with a diameter of 10 cm. A piece of AMRC fabric (about 1.3 gram) was placed on the MC solution in the crystallization dish. After soaking the fabric for 3 minutes, the wet fabric in the dish was lyophilized overnight. A very flexible patch was formed. The patch was further dried at room temperature under vacuum.

The AMRC/MC patch then was evaluated for hemostasis as set forth below. Results are provided in Table 1.

### Example 7:

### AMRC/CMC-Na porous patch preparation

One gram of sodium salt of carboxymethyl cellulose (CMC-Na, Type: 7M8SF Lot#: 77521 from Aqualon, Wlmington, DE) was dissolved in 99 grams of deionized water. After complete dissolution of the polymer, 10 grams of the Na-CMC solution was transferred into a crystallization dish with a diameter of 10 cm. A piece of AMRC fabric (about 1.3 gram) was placed on the CMC solution in the crystallization dish. After soaking the fabric for 3 minutes, the wet fabric in the dish was lyophilized overnight. A very flexible patch was formed. The patch was further dried at room temperature under vacuum.

The AMRC/CMC-Na patch then was evaluated for hemostasis as set forth below. Results are provided in Table 1.

### Example 8:

### AMRC/CMC-Na porous patch preparation

One gram of sodium salt of carboxymethyl cellulose (CMC-Na, Type: 7H4F Lot#: 79673 from Aqualon, Wilmington, DE) was dissolved in 99 grams of deionized water. After complete dissolution of the polymer, 10 grams of the Na-CMC solution was transferred into a crystallization dish with a diameter of 10 cm. A piece of AMRC fabric (about 1.3 gram) was placed on the CMC solution in the crystallization dish. After soaking the fabric for 3 minutes, the wet fabric in the dish was then lyophilized overnight. A very flexible patch was formed. The patch was further dried at room temperature under vacuum.

The AMRC/CMC-Na patch then was evaluated for hemostasis as set forth below. Results are provided in Table 1.

### Example 9:

### AMRC/HEC porous patch preparation:

One gram of hydroxyethyl cellulose (HEC, Ave. Mv; 720kD lot # 02808DU from Aldrich, Milwaukee, WI) was dissolved in 99 grams of deionized water. After complete dissolution of the polymer, 10 grams of the HEC solution was transferred into a crystallization dish with a diameter of 10 cm. A piece of AMRC fabric (about 1.3 gram) was placed on the HEC solution in the crystallization dish. After soaking the fabric in the solution for 3 minutes, the wet fabric in the dish was lyophilized overnight. A very flexible patch was formed. The patch was further dried at room temperature under vacuum.

The AMRC/HEC patch then was evaluated for hemostasis as set forth below. Results are provided in Table 1.

### Example 10:

### AMRC/HEC/Thrombin porous patch preparation

One gram of hydroxyethyl cellulose (HEC, Ave. Mv; 720kD lot # 02808DU from Aldrich, Milwaukee, WI) was dissolved in 99 grams of deionized water. After complete dissolution of the polymer, 20 ml of the MC solution was used to reconstitute thrombin in a vial (20,000 units). 2.5 ml of the cloudy solution was transferred into a crystallization dish. A piece of AMRC fabric (about 1 gram) was placed on the HEC solution in the crystallization dish. After soaking the fabric in the solution for 3 minutes, the wet fabric in the dish was lyophilized overnight. A very flexible patch was formed. The patch was further dried at room temperature under vacuum.

The AMRC/HEC/Thrombin porous patch then was evaluated for hemostasis as set forth below. Results are provided in Table 1.

### Example 11:

### AMRC/MC/Thrombin porous patch preparation

One gram of methyl cellulose (MC, Ave. Mn 63kD, lot# 06827ES from Aldrich) was dissolved in 99 grams of deionized water. After complete dissolution of the polymer, 20 ml of the MC solution was used to reconstitute thrombin in a vial (20,000 units). 2.5 ml of the cloudy solution was transferred into a crystallization dish. A piece of AMRC fabric (about 1 gram) was placed on the MC solution in the crystallization dish. After soaking the fabric in the solution for 3 minutes, the wet fabric in the dish was lyophilized overnight. A very flexible patch was formed. The patch was further dried at room temperature under vacuum.

The AMRC/MC/Thrombin porous patch then was evaluated for hemostasis as set forth below. Results are provided in Table 1.

### Example 12:

### AMRC/AMMC/Thrombin porous patch preparation:

One gram of aldehyde-modified methyl cellulose (AMMC) from Example 2 was dissolved in 99 grams of deionized water. After complete dissolution of the polymer, 20 ml of the AMMC solution was used to reconstitute thrombin in a vial (20,000 units). 2.5 ml of the cloudy solution was transferred into a crystallization dish. A piece of AMRC fabric (about 1 gram) was placed on the AMMC solution in the crystallization dish. After soaking the fabric in the solution for 3 minutes, the wet fabric in the dish was lyophilized overnight. A very flexible patch was formed. The patch was further dried at room temperature under vacuum.

### Example 13:

### AMRC/AMHEC/Thrombin porous patch preparation:

One gram of aldehyde-modified hydroxyethyl cellulose (AMHEC)(MW=90kD,from Aldrich) synthesized as perexample 3 was dissolved in 99 grams of deionized water. After complete dissolution of the polymer, 20 ml of the AMHEC solution was used to reconstitute thrombin in a vial (20,000 units). 2.5 ml of the cloudy solution was transferred into a crystallization dish. A piece of AMRC fabric (about 1 gram) was placed on the AMHEC solution in the crystallization dish. After soaking the fabric in the solution for 3 minutes, the wet fabric in the dish was lyophilized overnight. A very flexible patch was formed. The patch was further dried at room temperature under vacuum.

The AMRC/AMHEC/Thrombin porous patch then was evaluated for hemostasis as set forth below. Results are provided in Table 1.

### Example 14:

### Hemostatic performance of different materials in porcine splenic incision model

A porcine spleen incision model was used for hemostasis evaluation of different materials. The materials were cut into 2.5 cm X 1.5 cm rectangles. A linear incision of 1.5 cm with a depth of 0.3 cm was made with a surgical blade on a porcine spleen. After application of the test article, digital tamponade was applied to the incision for 2 minutes. The hemostasis was then evaluated. Additional applications of digital tamponade for 30 seconds each time were used until complete hemostasis was achieved. Fabrics failing to provide hemostasis within 12 minutes were considered to be failures. Table 1 lists the results of the evaluation.

### Example 15:

### Hemostatic performance of different materials in a porcine splenic incision model with tamponade for 30 seconds

A porcine spleen incision model was used for hemostasis evaluation of different materials. The materials were cut into 2.5 cm X 1.5 cm rectangles. A linear incision of 1.5 cm with a depth of 0.3 cm was made with a surgical blade on porcine spleen. After application of the test article, digital tamponade was applied to the incision for 30 seconds. The hemostasis evaluation was then performed. Additional applications of digital tamponade for 30 seconds each time were used until complete hemostasis was achieved. Table 1 lists the results of the evaluation.

**Table 1**

| Hemostatic performance of Aldehyde-Modified Regenerated Cellulose (AMRC) Based-Materials | | |
|---|---|---|
| | 2 min tamponade | 30 second tamponade |
| Sample | Time to Hemostasis (Seconds) | Time to Hemostasis (Seconds) |
| Example 1 | 187 (n=11) | |
| Example 4 | 370 (n=2) | |
| Example 5 | 308 (n=2) | |
| Example 6 | 285 (n=1) | |
| Example 7 | 582 (n=2) | |
| Example 8 | 120 (n=3) | 230 (n=2) |
| Example 9 | 187 (n=3) | 253 (n=2) |
| Example 10 | | 73 (n=3) |
| Example 11 | | 30 (n=3) |
| Example 13 | | 47 (n=3) |
| Surgical gauze Negative Control | >720 | >720 |

As indicated from the results, wound dressings of the present invention achieve effective hemostasis. In particular, when higher molecular weight water-soluble polymers (CMC-Na and HEC) were used, the corresponding patches achieved better time to hemostasis. Also as indicated from the results, wound dressings of the present invention having hemostatic agents, e.g. thrombin, bound there to achieve even faster time to hemostasis.

## Claims

1. A hemostatic wound dressing, comprising:
a fabric, said fabric comprising a first wound-contacting surface and a second surface opposing said wound-contacting surface, said fabric comprising fibers and having flexibility, strength and porosity effective for use as a hemostat, said fibers comprising a biocompatible, aldehyde-modified polysaccharide; and
a porous, polymeric matrix applied to said wound-contacting surface and dispersed at least partially through said fabric, said porous polymeric matrix comprising a biocompatible, water-soluble or water-swellable polymer,
wherein said wound dressing is hemostatic.

2. The wound dressing of claim 1 wherein said aldehyde-modified polysaccharide is selected from the group consisting of cellulose, cellulose derivatives, chitin, carboxymethyl chitin, hyaluronic acid, salts of hyaluronic acid, alginate, alginic acid, propylene glycol alginate, glycogen, dextran, dextran sulfate, curdlan, pectin, pullulan, xanthan, chondroitin, chondroitin sulfates, carboxymethyl dextran, carboxymethyl chitosan, heparin, heparin sulfate, heparan, heparan sulfate, dermatan sulfate, keratin sulfate, carrageenans, chitosan, starch, amylose, amylopectin, poly-N-glucosamine, polymannuronic acid, polyglucuronic acid, polyguluronic acid and derivatives of the above.

3. The wound dressing of claim 2 wherein said aldehyde-modified polysaccharide comprises an amount of aldehyde effective to render the polysaccharide biodegradable.

4. The wound dressing of claim 3 wherein said aldehyde-modified polysaccharide is selected from the group consisting of starch, dextran, pectin, alginate, chitin, chitosan, glycogen, amylose, amylopectin, cellulose and cellulose derivatives thereof.

5. The wound dressing of claim 4 wherein said aldehyde-modified polysaccaride comprises aldehyde-modified regenerated polysaccharide.

6. The wound dressing of claim 5 wherein said aldehyde-modified polysaccharide comprises aldehyde-modified regenerated cellulose comprising repeating units of structure II, 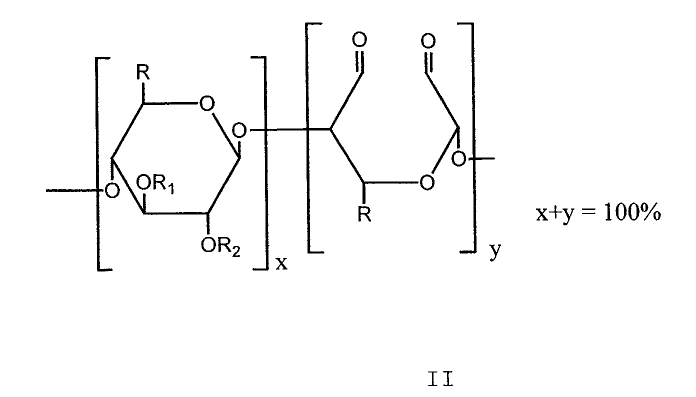 wherein x plus y equals 100 percent, x ranges from about 95 to about 5 percent, and
y ranges from about 5 to about 95 percent and R is CH₂OH, and R₁ and R₂ are H.

7. The wound dressing of claim 1 wherein said aldehyde-modified polysaccharide is essentially free of carboxylic acid.

8. The wound dressing of claim 6 wherein said aldehyde-modified cellulose is essentially free of carboxylic acid.

9. The wound dressing of claim 1 further comprising a hemostatic agent.

10. The wound dressing of claim 9 wherein said hemostatic agent is synthetic, recombinant or naturally occurring.

11. The wound dressing of claim 10 wherein said hemostatic agent is selected from the group consisting prothrombin, thrombin, fibrinogen, fibrin, fibronectin, heparinase, Factor X/Xa, Factor VII/VIIa, Factor IX/IXa, Factor XI/XIa, Factor XII/XIIa, tissue factor, batroxobin, ancrod, ecarin, von Willebrand Factor, collagen, elastin, albumin, gelatin, platelet surface glycoproteins, vasopressin, vasopressin analogs, epinephrine, selectin, procoagulant venom, plasminogen activator inhibitor, platelet activating agents and synthetic peptides having hemostatic activity and derivatives of the above.

12. The wound dressing of claim 11 comprising from about 0.001 to about 50 percent by weight of said hemostatic agent.

13. The wound dressing of claim 8 wherein said water-soluble or water-swellable polymer is selected from the group consisting of non-acidic methyl cellulose, hydroxyalkyl cellulose, water-soluble chitosan, salts of carboxymethyl carboxyethyl cellulose, chitin, salts of hyaluronic acid, alginate, propylene glycol alginate, glycogen, dextran, carrageenans, chitosan, starch, amylose, poly-N-glucosamine and derivatives of the above and the aldehyde-modified derivatives thereof, said wound dressing comprising from about 0.001 to about 50 percent by weight of said hemostatic agent selected from the group consisting of thrombin, fibrin and fibrinogen.

14. The wound dressing of claim 13 comprising from about 0.001 to about 1 percent thrombin as the hemostatic agent.

15. The wound dressing of claim 13 comprising from about 0.1 to about 50 percent by weight of fibrinogen as the hemostatic agent.

16. The wound dressing of claim 13 comprising from about 0.1 to about 50 percent by weight of fibrin as the hemostatic agent.

17. The wound dressing of claim 9 comprising said hemostatic agent dispersed at least partially through said porous, polymeric matrix.

18. The wound dressing of claim 9 comprising said hemostatic agent dispersed substantially homogenously through said porous, polymeric matrix.

19. The wound dressing of claim 9 wherein said first wound-contacting surface of said fabric comprises said hemostatic agent.

20. The wound dressing of claim 9 comprising said hemostatic agent dispersed substantially homogenously through said fabric.

21. The wound dressing of claim 9 wherein said hemostatic agent is dispersed at least partially through said fabric.

22. The wound dressing of claim 1 wherein said water-soluble or water-swellable polymer is selected from the group consisting of polysaccharides, polyacrylic acids, polymethacrylic acids, polyamines, polyimines, polyamides, polyesters, polyethers, polynucleotides, polynucleic acids, polypeptides, proteins, poly (alkylene oxides), polythioesters, polythioethers, polyvinyls and polymers comprising lipids.

23. The wound dressing of claim 22 wherein said water-soluble or water-swellable polymer is a polysaccharide.

24. The wound dressing of claim 22 wherein said polysaccharide is selected from the group consisting of cellulose, cellulose derivatives, chitin, carboxymethyl chitin, hyaluronic acid, salts of hyaluronic acid, alginate, alginic acid, propylene glycol alginate, glycogen, dextran, dextran sulfate, curdlan, pectin, pullulan, xanthan, chondroitin, chondroitin sulfates, carboxymethyl dextran, carboxymethyl chitosan, heparin, heparin sulfate, heparan, heparan sulfate, dermatan sulfate, keratin sulfate, carrageenans, chitosan, starch, amylose, amylopectin, poly-N-glucosamine, polymannuronic acid, polyglucuronic acid, polyguluronic acid and derivatives of the above.

25. The wound dressing of claim 1 wherein said porous polymeric matrix comprises lyophilized sodium carboxymethyl cellulose.

26. The wound dressing of claim 25 wherein the weight ratio of said lyophilized sodium carboxymethyl cellulose to said fabric is from about 1:99 to about 20:80.

27. The wound dressing of claim 1 wherein said porous polymeric matrix is dispersed substantially homogeneously through said fabric.

28. The wound dressing of claim 1 wherein said porous polymeric matrix is dispersed through said fabric in a gradient, whereby the concentration of the water-soluble or water-swellable polymer adjacent said first wound-contacting surface is greater than the concentration of the water-soluble or water-swellable polymer adjacent said second opposing surface.

29. A fabric, comprising:
a first surface and a second surface opposing said first surface, said fabric comprising fibers comprising a biocompatible, aldehyde-modified polysaccharide.

30. The fabric of claim 29 wherein said fabric is hemostatic.

31. The fabric of claim 30 wherein said fibers comprise aldehyde-modified regenerated polysaccharide and said porous polymeric matrix comprises a lyophilized polysaccharide selected from the group consisting of cellulose, cellulose derivatives, chitin, carboxymethyl chitin, hyaluronic acid, salts of hyaluronic acid, alginate, alginic acid, propylene glycol alginate, glycogen, dextran, dextran sulfate, curdlan, pectin, pullulan, xanthan, chondroitin, chondroitin sulfates, carboxymethyl dextran, carboxymethyl chitosan, heparin, heparin sulfate, heparan, heparan sulfate, dermatan sulfate, keratin sulfate, carrageenans, chitosan, starch, amylose, amylopectin, poly-N-glucosamine, polymannuronic acid, polyglucuronic acid, polyguluronic acid and derivatives of the above.

32. The fabric of claim 29 wherein said fabric is essentially free of carboxylic acid.

33. The fabric of claim 29 further comprising a hemostatic agent.

34. The fabric of claim 30 further comprising a hemostatic agent.

35. A process for making a wound dressing: comprising,
providing a solution having substantially dissolved therein a water-soluble or water-swellable biocompatible polymer,
providing a fabric having a top surface and a bottom surface opposing said top surface, said fabric comprising fibers and having flexibility, strength and porosity effective for use as a hemostat, said fibers comprising an aldehyde-modified polysaccharide,
contacting said solution with said fabric under conditions effective to distribute said solution through said fabric,
lyophilizing said fabric having said solution distributed there through, thereby providing a porous, polymeric matrix comprising said water-soluble or water-swellable polymer dispersed through said fabric.

36. The process of claim 35 wherein said fabric is knitted.

37. The process of claim 36 wherein said fibers comprise an aldehyde-modified regenerated cellulose.

38. The process of claim 37 wherein said porous polymeric matrix comprises a polymer selected from the group consisting of polysaccharides, polyacrylic acids, polymethacrylic acids, polyamines, polyimines, polyamides, polyesters, polyethers, polynucleotides, polynucleic acids, polypeptides, proteins, poly (alkylene oxides), polythioesters, polythioethers, polyvinyls, polymers comprising lipids and derivatives of the above.

39. The process of claim 38 wherein said porous polymeric matrix comprises a polysaccharide selected from the group consisting of cellulose, cellulose derivatives, chitin, carboxymethyl chitin, hyaluronic acid, salts of hyaluronic acid, alginate, alginic acid, propylene glycol alginate, glycogen, dextran, dextran sulfate, curdlan, pectin, pullulan, xanthan, chondroitin, chondroitin sulfates, carboxymethyl dextran, carboxymethyl chitosan, heparin, heparin sulfate, heparan, heparan sulfate, dermatan sulfate, keratin sulfate, carrageenans, chitosan, starch, amylose, amylopectin, poly-N-glucosamine, polymannuronic acid, polyglucuronic acid, polyguluronic acid and derivatives of the above.

40. The process of claim 39 wherein said porous polymeric matrix comprises sodium carboxymethyl cellulose, wherein the weight ratio of said sodium carboxymethyl cellulose to said fabric is from about 1:99 to about 20:80.

41. The process of claim 35 further comprising an effective amount of a hemostatic agent admixed with said solution.

42. A method of providing hemostasis to a wound, comprising:
applying to a wound a hemostatic wound dressing, comprising:
a fabric, said fabric comprising a first wound-contacting surface and a second surface opposing said wound-contacting surface, said fabric comprising fibers and having flexibility, strength and porosity effective for use as a hemostat, said fibers comprising a biocompatible, aldehyde-modified polysaccharide; and
a porous, polymeric matrix applied to said wound-contacting surface and dispersed at least partially through said fabric, said porous, polymeric matrix comprising a biocompatible, water-soluble or water-swellable polymer,
wherein said wound dressing is hemostatic.

43. The method of claim 42 wherein said aldehyde-modified polysaccharide is selected from the group consisting of cellulose, cellulose derivatives, chitin, carboxymethyl chitin, hyaluronic acid, salts of hyaluronic acid, alginate, alginic acid, propylene glycol alginate, glycogen, dextran, dextran sulfate, curdlan, pectin, pullulan, xanthan, chondroitin, chondroitin sulfates, carboxymethyl dextran, carboxymethyl chitosan, heparin, heparin sulfate, heparan, heparan sulfate, dermatan sulfate, keratin sulfate, carrageenans, chitosan, starch, amylose, amylopectin, poly-N-glucosamine, polymannuronic acid, polyglucuronic acid, polyguluronic acid and derivatives of the above.

44. The method of claim 43 wherein said aldehyde-modified polysaccharide comprises an amount of aldehyde effective to render the polysaccharide biodegradable.

45. The method of claim 44 wherein said aldehyde-modified polysaccaride comprises aldehyde-modified regenerated polysaccharide.

46. The wound dressing of claim 45 wherein said aldehyde-modified polysaccharide comprises aldehyde-modified regenerated cellulose comprising repeating units of structure II, 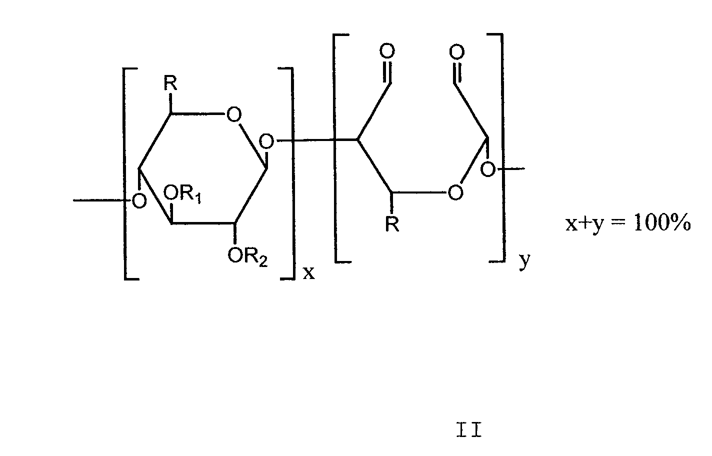 wherein x plus y equals 100 percent, x ranges from about 95 to about 5 percent, and
y ranges from about 5 to about 95 percent and R is CH₂OH, and R₁ and R₂ are H.

47. The method of claim 42 wherein said aldehyde-modified polysaccharide is essentially free of carboxylic acid.

48. The method of claim 46 wherein said aldehyde-modified cellulose is essentially free of carboxylic acid.

49. The method of claim 42 wherein said wound dressing further comprises a hemostatic agent.

50. The method of claim 49 wherein said hemostatic agent is selected from the group consisting prothrombin, thrombin, fibrinogen, fibrin, fibronectin, heparinase, Factor X/Xa, Factor VII/VIIa, Factor IX/IXa, Factor XI/XIa, Factor XII/XIIa, tissue factor, batroxobin, ancrod, ecarin, von Willebrand Factor, collagen, elastin, albumin, gelatin, platelet surface glycoproteins, vasopressin, vasopressin analogs, epinephrine, selectin, procoagulant venom, plasminogen activator inhibitor, platelet activating agents and synthetic peptides having hemostatic activity and derivatives of the above.

51. The method of claim 42 wherein said porous, polymeric matrix comprises a polymer selected from the group consisting of polysaccharides, polyacrylic acids, polymethacrylic acids, polyamines, polyimines, polyamides, polyesters, polyethers, polynucleotides, polynucleic acids, polypeptides, proteins, poly (alkylene oxides), polythioesters, polythioethers, polyvinyls, polymers comprising lipids and derivatives of the above.
